# EUROPEAN PATENT APPLICATION

(11) **EP 0 978 512 A1**
(43) Date of publication of application: **09.02.2000**
(21) Application number: 98401944.8
(22) Date of filing: 29.07.1998
(51) Int. Cl.: C07D 295/088, C07C 211/08, C07D 211/04, C07D 295/185, C07D 211/62, C07D 211/70, C07D 207/20, A61K 31/13, A61K 31/40, A61K 31/445, A61K 31/495

(54) **Non-imidazole aryloxy (or arylthio) alkylamines as histamine H3-receptor antagonists and their therapeutic applications**

(71) Applicant: SOCIETE CIVILE BIOPROJET, F-75003 Paris (FR)
(72) Inventor: Schwartz, Jean-Charles, 75014 Paris (FR); Arrang, Jean-Michel, 91419 Dourdan (FR); Garbarg, Monique, 75017 Paris (FR); Lecomte, Jeanne-Marie, 75003 Paris (FR); Ligneau, Xavier, 75013 Paris (FR); Schunack, Walter G., 14129 Berlin (DE); Stark, Holger, 12157 Berlin (DE); Ganellin, Charon Robin, Welwyn, Hert AL6 OTD (GB); Leurquin, Fabien, London E2 6DZ (GB); Sigurd, Elz, 12107 Berlin (DE)
(74) Representative: Le Guen, Gérard

(57) **Abstract**

Compounds of formula (I): 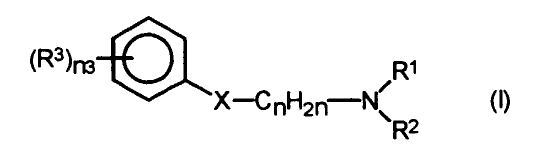 and their use for preparing medicaments acting as antagonists at the H₃-receptors of histamine.

## Description

The present invention relates to novel aryloxy (or arylthio) alkylamines, to their preparation and to their therapeutic applications.

Antagonists of histamine H₃ receptor are known especially to increase synthesis and release of cerebral histamine. Through this mechanism, they induce an extended wakefullness, an improvement in cognitive processes, a reduction in food intake and a normalization of vestibular reflexes (Schwartz et al., Physiol. Rev., 1991, 71; 1-51).

Whence these agents are potentially useful in several central nervous system disorders such as Alzheimer disease, mood and attention alterations, cognitive deficits in psychiatric pathologies, obesity, vertigo and motion sickness.

All the H₃ receptor antagonist compounds known so far resemble histamine in possessing an imidazole ring (Ganellin et al., Ars Pharmaceutica, 1995, 36:3, 455-468; Stark et al., Drug of the Future, 1996, 21(5), 507-520).

Nevertheless, such imidazole derivatives may show drawbacks such as poor blood-brain barrier penetration and/or some hepatic and ocular toxicities. These drawbacks, which can prevent their therapeutic development, appear to be linked to the presence of an imidazole ring substituted by a hydrophobic chain.

Attempts to develop H₃ receptor antagonists without an imidazole ring have up to now been unsuccessful, the compounds being of low potency.

In this respect, non-imidazole compounds such as betahistine (J-M. Arrang et al., Eur. J. Pharmacol. 1985, 111: 72-84), phencyclidine (J-M. Arrang et al., Eur. J. Pharmacol. 1988, 157: 31-35), dimaprit (J-C Schwartz et al., Agents Actions 1990, 30: 13-23), clozapine (M. Kathmann et al., Psychopharmacology 1994, 116: 464-468), and sesquiterpenes (M. Takigawa et al., JP 06 345 642 (20 Dec 1994)) were suggested to display H₃-receptor antagonism but all these compounds have only very low potency.

The present invention provides new compounds, the structure of which do not contain an imidazole moiety, which are useful as histamine H₃-receptor antagonists while avoiding the above-mentioned drawbacks of the known H₃-antagonists.

The compounds of the invention have the following general formula (I): 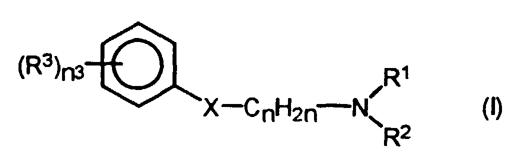 in which:
― CₙH₂ₙ is a linear or branched hydrocarbon chain with n ranging from 2 to 8;
― X is an oxygen or sulfur atom;
― R¹ and R² may be identical or different and represent each independently
   - a lower alkyl or cycloalkyl, or taken together with the nitrogen atom to which they are attached,
   - a saturated nitrogen-containing ring 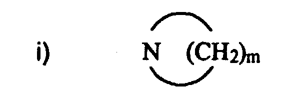 with m ranging from 4 to 7, or
   - an unsaturated nitrogen-containing ring 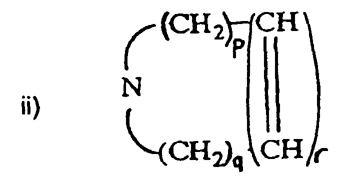 with p, q and r being 1 to 3 independently, such nitrogen-containing ring i) or ii) being unsubstituted or substituted by one or more lower alkyl or cycloalkyl, or carboalkoxy groups, or
   - a morpholino group, or
   - a N-substituted piperazino group: 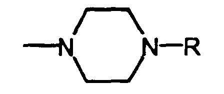 with R being a lower alkyl, an alkanoyl or an optionally substituted phenyl group;
― n₃ is an integer from 0 to 5;
― R³ represents each independently
   - a halogen atom,
   - a lower alkyl or cycloalkyl, a trifluoromethyl, aryl, alkoxy, aryloxy, nitro, formyl, alkanoyl, aroyl, arylalkanoyl, amino, carboxamido, cyano, alkyloximino, aryloximino, α-hydroxyalkyl, alkenyl, alkynyl, sulphamido, sulfamoyl, carboxamide, carboalkoxy, arylalkyl or oxime group,
   - or taken together with the carbon atoms of the phenyl ring to which it is fused, a 5- or 6-membered saturated or unsaturated ring or a benzene ring.

The invention also relates to the addition salts which the compounds form with pharmaceutically acceptable acids. The pharmaceutically acceptable salts comprise the nontoxic salt of inorganic or organic acids. Examples of these salts include the hydrochloride, the hydrobromide or the hydrogen maleate or hydrogen oxalate.

The present invention also encompasses the hydrates of the compounds, the hydrated salts of these compounds and the polymorphic crystalline structures. When the compounds can exist in one or a number of isomeric forms according to the number of asymmetric centres in the molecule, the invention relates both to all the optical isomers and to their racemic modifications and the corresponding diastereoisomers. The separation of the diastereoisomers and/or of the optical isomers can be carried out according to methods known per se.

According to the invention, lower alkyl or cycloalkyl is intended to mean a linear or branched alkyl group containing from 1 to 6 carbon atoms, or a saturated carbocycle containing 3 to 6 carbon atoms.

Typically examples of lower alkyl are methyl, ethyl, propyl, isopropyl and butyl groups.

A preferred group of compounds according to the invention comprises those with R¹ and R² representing independently a lower alkyl group, especially an ethyl group.

Preferred compounds are also those of formula (I) in which R¹ and R² taken together with the nitrogen atom to which they are attached, form a saturated nitrogen-containing ring: 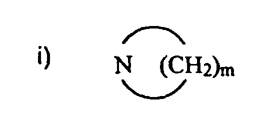 especially with m being 4, 5 or 6, optionally substituted with an alkyl group, preferably a methyl group.

Another preferred group of compounds comprises compounds (I) in which R¹ and R² taken together with the nitrogen atom to which they are attached, form an unsaturated nitrogen-containing ring: 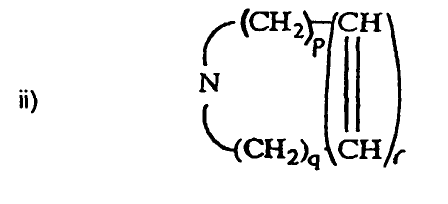 especially with p, q, and r being 1 or 2. In this group, more preferred compounds are those with p being 2 and q and r each being 1.

Typical example of -NR¹R² representing a N-substituted piperazino group is N-acetylpiperazino.

A preferred group of compounds according to the invention is the group composed of compounds of formula (I) in which X is an oxygen atom.

Another preferred group of compounds comprises compounds (I) in which -CₙH₂ₙ- is a linear chain -(CH₂)ₙ- with n being as previously defined.

Preferred compounds are also those with n varying from 3 to 5, and with n being more preferably 3.

A sub-class of compounds according to the invention comprises the compounds of formula (I) with n₃ being zero that is those having an unsubstituted phenyl moiety.

Another group of compounds according to the invention is composed of compounds containing one or more substituents R³ which may be identical or different. In this group, the compounds having a mono- or di-substituted (n₃ = 1 or 2) phenyl moiety are preferred and those mono-substituted with one group R³ as defined above in para-position are particularly preferred.

Among these compounds, (n₃ being 1) R³ is preferably a halogen atom or a cyano, nitro, alkanoyl, alkyloximino or α-hydroxyalkyl group.

Still more preferred compounds are those with R³ being CN, NO₂, COCH₃, COC₂H₅, H₃C-C=N-OH, H₃C-CH-OH.

R³ being a halogen atom may be advantageously selected from fluorine, chlorine and bromine.

R³ being an aryl group, may be especially a phenyl group.

In the other substituents R³, the aryl moiety is advantageously a phenyl moiety.

R³ being an aryloxy group may be especially a phenoxy group.

According to the invention, alkanoyl is intended to mean a group containing an alkyl moiety as defined above.

Typical examples of R³ being an alkanoyl, aroyl or arylalkanoyl group are acetyl, butyryl and propionyl groups, benzoyl group or phenylacetyl group.

Typical examples of R³ forming together with the carbon atoms of the phenyl ring to which it is fused, a saturated ring leads to 5,6,7,8-tetrahydronaphthyl or forming a benzene ring leads to a naphthyl moiety.

According to the invention, alkenyl or alkynyl group may contain advantageously from 1 to 8 carbon atoms, in particular from 1 to 6 carbon atoms and preferably 1 to 4 carbon atoms.

In carboalkoxy, carboxyamido or carboxamide groups, the hydrocarbon chain is saturated, linear or branched and contains an alkyl moiety as defined above.

In alkoxy, alkyloximino, arylalkyl or α-hydroxyalkyl group, the alkyl moiety is as previously defined also.

Particularly preferred compounds are:
1-(5-phenoxypentyl)-piperidine
1-(5-phenoxypentyl)-pyrrolidine
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(4-phenoxybutyl)-pyrrolidine
1-(6-phenoxyhexyl)-pyrrolidine
1-(5-phenylthiopentyl)-pyrrolidine
1-(4-phenylthiobutyl)-pyrrolidine
1-(3-phenoxypropyl)-pyrrolidine
1-[5-(3-nitrophenoxy)-pentyl]-pyrrolidine
1-[5-(4-fluorophenoxy)-pentyl]-pyrrolidine
1-[5-(4-nitrophenoxy)-pentyl]-3-methyl-piperidine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
N-[3-(4-nitrophenoxy)-propyl]-diethylamine
N-[3-(4-cyanophenoxy)-propyl]-diethylamine
1-[5-(4-benzoylphenoxy)-pentyl]-pyrrolidine
1-{5-[4-(phenylacetyl)-phenoxy]-pentyl}-pyrrolidine
N-[3-(4-acetylphenoxy)-propyl]-diethylamine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-[4-(1-hydroxyethyl)-phenoxy]-pentyl}-pyrrolidine
1-[5-(4-cyanophenoxy)-pentyl]-diethylamine
1-[5-(4-cyanophenoxy)-pentyl]-piperidine
N-[5-(4-cyanophenoxy)-pentyl]-dimethylamine
N-[2-(4-cyanophenoxy)-ethyl]-diethylamine
N-[3-(4-cyanophenoxy)-propyl]-dimethylamine
N-[4-(4-cyanophenoxy)-butyl]-diethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine

More preferred compounds are:
1-[5-(4-nitrophenoxy)-pentyl]-pyrrolidine
N-[3-(4-cyanophenoxy)-propyl]-diethylamine
N-[3-(4-acetylphenoxy)-propyl]-diethylamine
1-{5-[4-(1-hydroxyethyl)-phenoxy]-pentyl)-pyrrolidine
N-[4-(4-cyanophenoxy)-butyl]-diethylamine
1-[3-(4-cyanophenoxy)-propyl]-piperidine
N-[3-(4-cyanophenoxy)-propyl]-hexamethyleneimine
N-3-[4-(1-hydroxyethyl)-phenoxy]-propyl-diethylamine
4-(3-diethylaminopropoxy)-acetophenone-oxime
1-[3-(4-acetylphenoxy)-propyl]-3-methyl-piperidine
1-[3-(4-acetylphenoxy)-propyl]-4-methyl-piperidine
1-[3-(4-propionylphenoxy)-propyl]-piperidine

Compounds of formula (I) in which:
- -NR¹R² is a pyrrolidinyl group, CₙH₂ₙ is a linear chain -(CH₂)ₙ- and n₃ is zero, X being an oxygen atom with n ranging from 3 to 5, or X being a sulfur atom with n being 4 or 5;
- -NR¹R² is a piperidinyl group, CₙH₂ₙ is a linear chain -(CH₂)ₙ- and X is an oxygen atom, n₃ being zero with n being 2, 5 or 8 or n₃ being 1 with R³ being 4-CN and n being 5;
- -NR¹R² is a diethylamine group, X is an oxygen atom, CₙH₂ₙ is a linear chain -(CH₂)ₙ- and n₃ is 1, R³ being 4-NO₂ or 4-COCH₃ with n being 3 or R³ being 4-CN with n being 2 to 4;
- -NR¹R² is a dimethylamine group, X is an oxygen atom, CₙH₂ₙ is a linear chain -(CH₂)ₙ- and n³ is 1, R³ being 4-CN with n being 3,
are known in the art.

A subject of the invention is thus the use of these compounds as antagonists at the histamine H₃-receptors, in particular to prepare medicaments acting as H₃-antagonists intended for the treatments detailed below.

The compounds according to the invention may be prepared according to one of the following schemes 1-5:

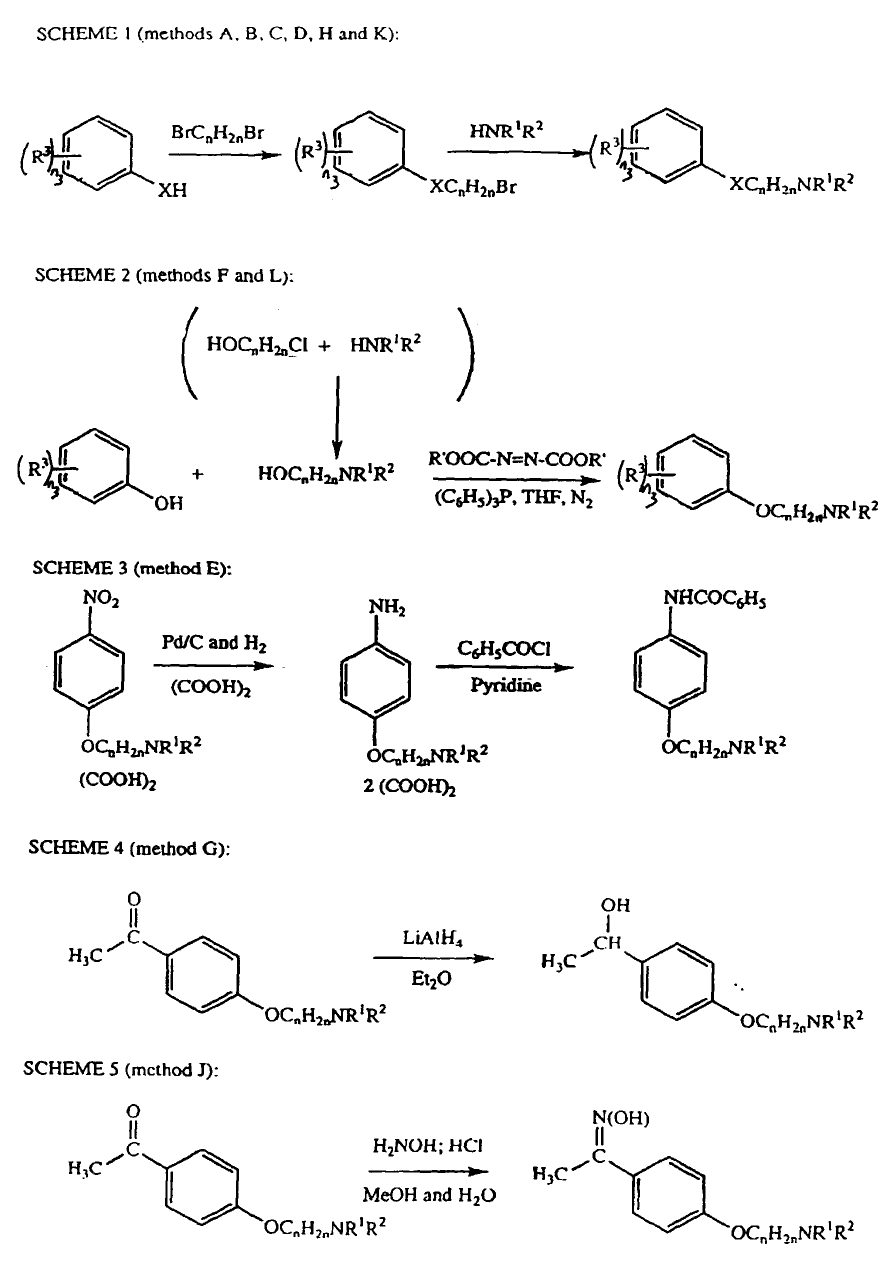

In these schemes, R¹, R², R³, X and n are as defined in general formula (I).

Me and Et are intended to mean methyl and ethyl.

Detailed synthesis procedures are given in the examples.

The compounds of formula (I) according to the invention have antagonistic properties at the histamine H₃-receptors. They cause an increase in synthesis and release of cerebral histamine.

This property makes the compounds of the invention useful derivatives in human or veterinary medicine.

Their therapeutical applications are those known for H₃-antagonist compounds and especially relate to the central nervous system disorders such as Alzheimer disease, mood and attention alterations, cognitive deficits in psychiatric pathologies, obesity, vertigo and motion sickness.

Therefore, the compounds of formula (I) according to the invention are advantageously used as active ingredient of medicaments which act as an antagonist of H₃-receptors of histamine, in particular of medicaments having psychotropic effects, promoting wakefullness, attention, memory and improving mood, in treatment of pathologies such as Alzheimer disease and other cognitive disorders in aged persons, depressive or simply asthenic states.

Their nootropic effects can be useful to stimulate attention and memorization capacity in healthy humans.

In addition, these agents can be useful in treatment of obesity, vertigo and motion sickness.

It can also be useful to associate the compounds of the invention with other psychiatric agents such as neuroleptics to increase their efficiency and reduce their side effects.

Application in certain form of epilepsy is also foreseen.

Their therapeutic applications involve also peripheral organs mainly a stimulant of secretions or gastro-intestinal motricity.

The compounds of the invention are particularly useful for the treatment of CNS disorders of aged persons.

The present invention also relates to medicaments having the above-mentioned effects comprising as active ingredient, a therapeutically effective amount of a compound of formula (I).

The present invention also relates to pharmaceutical compositions containing as active ingredient, a therapeutically effective amount of a compound (I) together with a pharmaceutically acceptable vehicle or excipient.

The medicaments or pharmaceutical compositions according to the invention can be administered via oral, parenteral or topical routes, the active ingredient being combined with a therapeutically suitable excipient or vehicle.

According to the invention, oral administration is advantageously used.

Another subject of the present invention is the use of the compounds of formula (I) for the preparation of H₃-antagonist medicaments according to the above-mentioned forms.

The invention further relates to the use of the compounds of formula (I) for preparing medicaments having the pre-cited effects.

Still another subject of the invention is a method for the treatment of precited ailments comprising administering a therapeutically effective dose of a compound (I), optionally in combination with a therapeutically acceptable vehicle or excipient.

For each of the above-indications, the amount of the active ingredient will depend upon the condition of the patient.

However, a suitable effective dose will be in general in the range of from 10 to 500 mg per day and of from 1 to 10 mg/day for particularly active compounds.

These doses are given on the basis of the compound and should be adapted for the salts, hydrates or hydrated salts thereof.

The invention is now illustrated by the following examples.

### EXAMPLES

The structure of the synthesized compounds and their method of preparation as well as their melting point, recrystalisation solvant and elemental analysis are summarized in the following Table I:

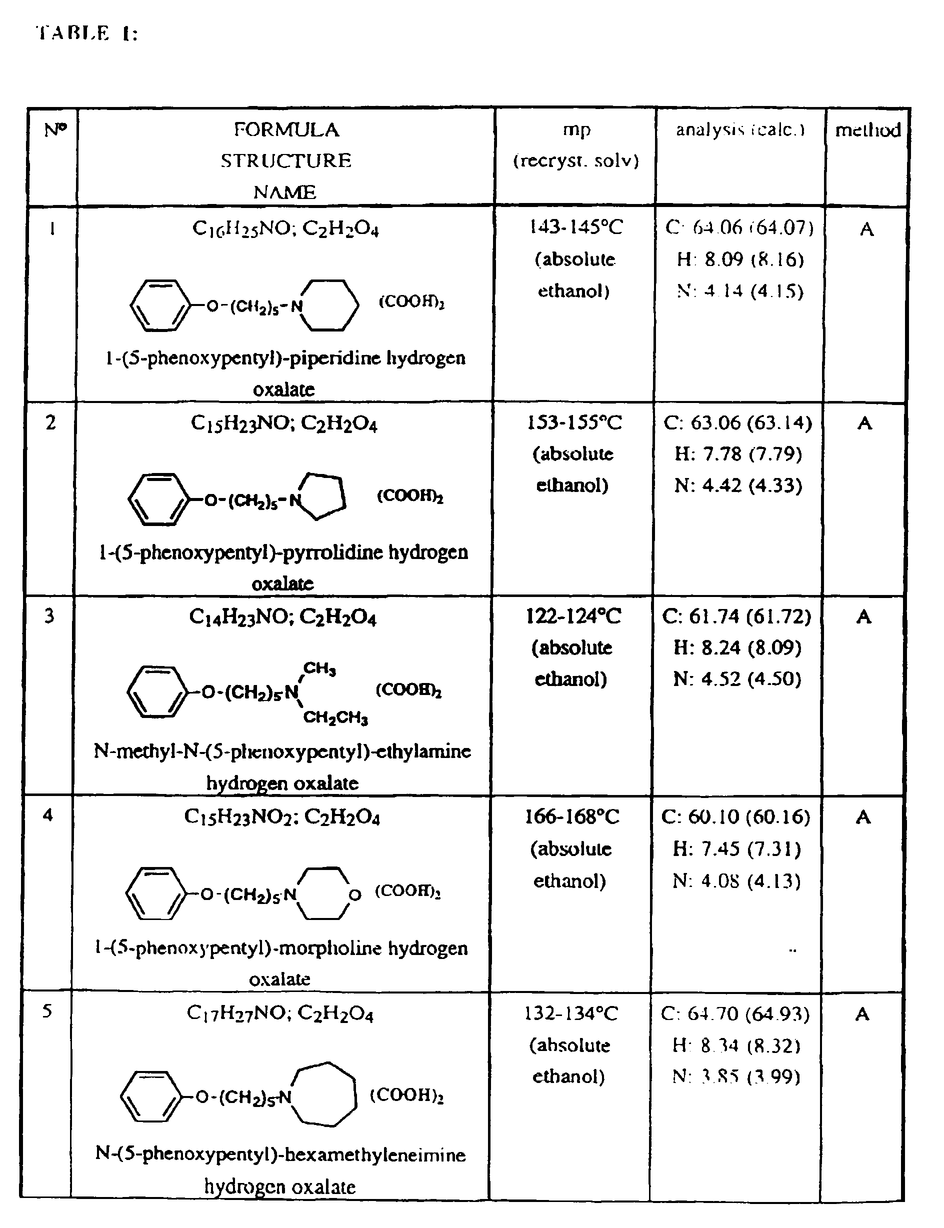

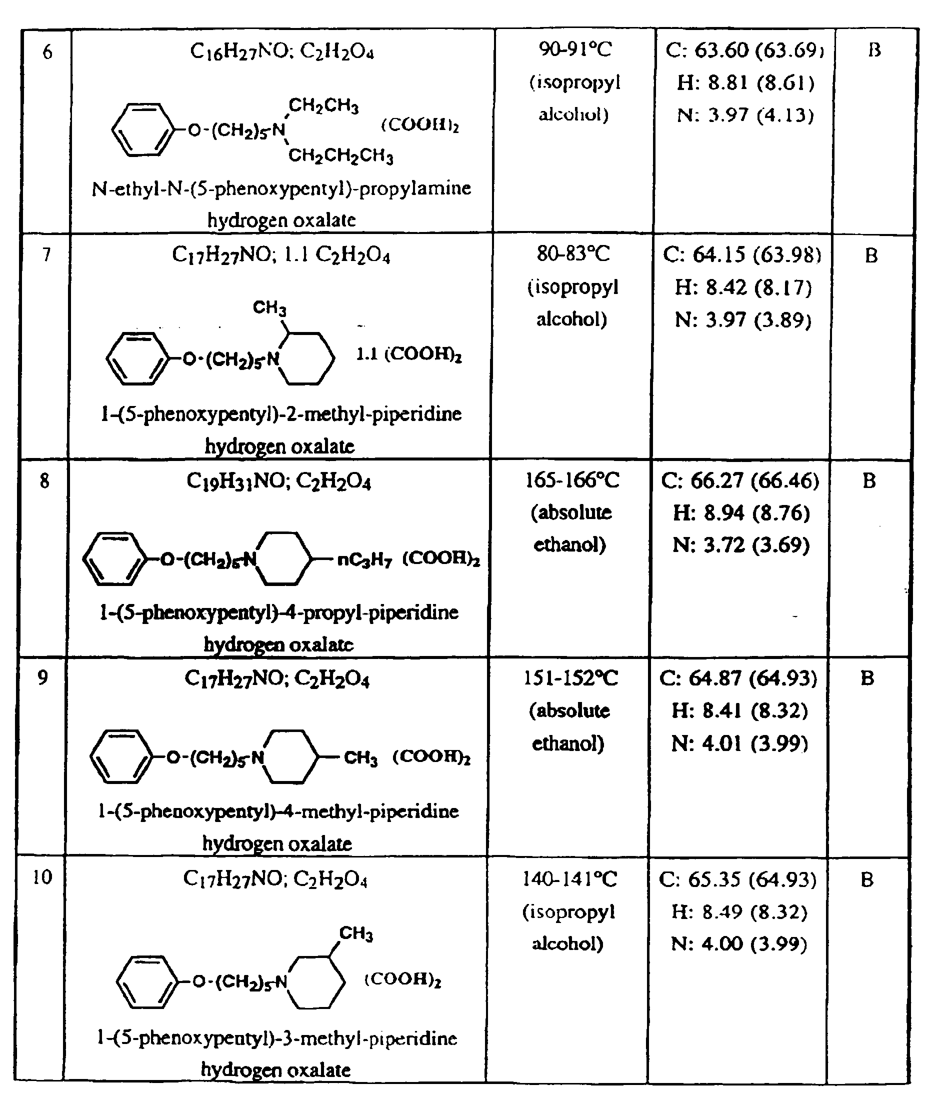

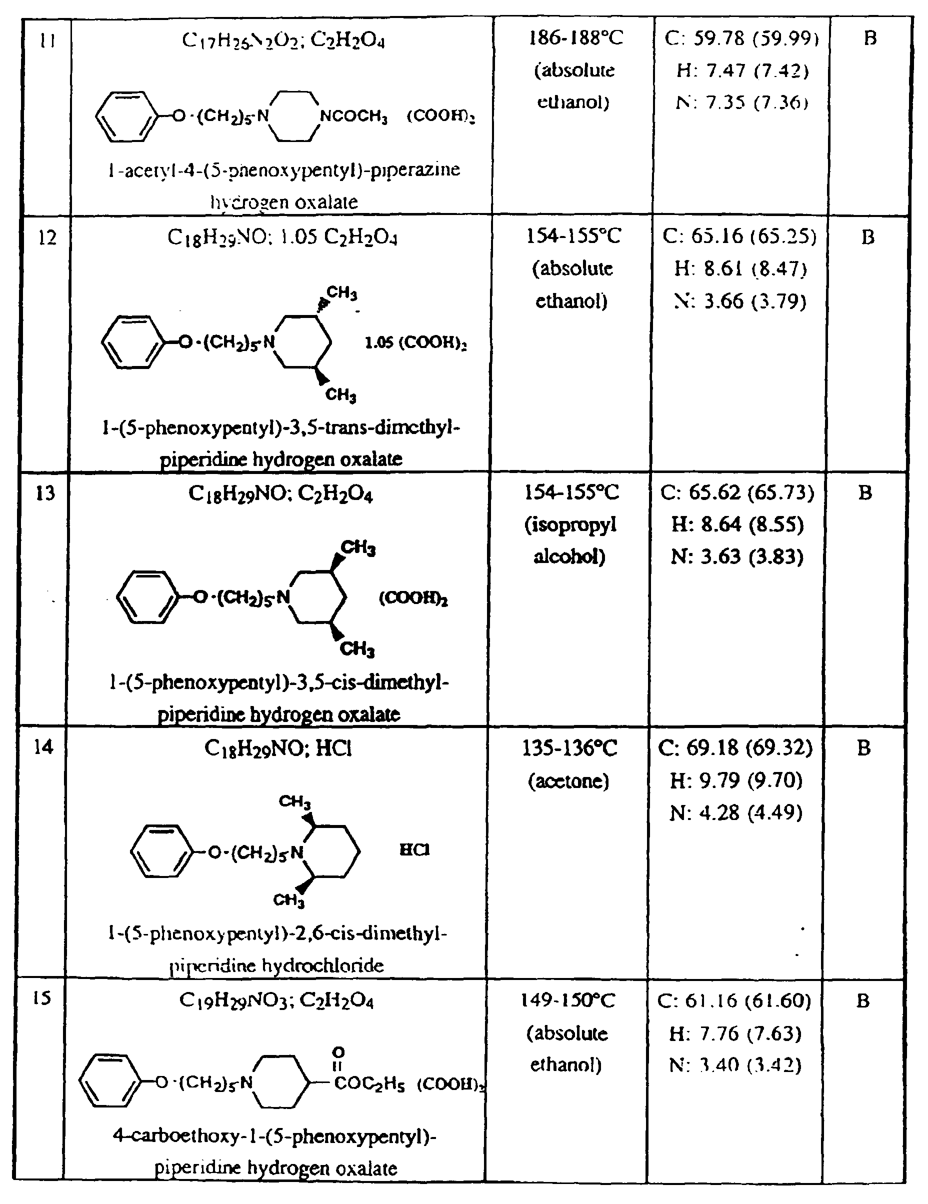

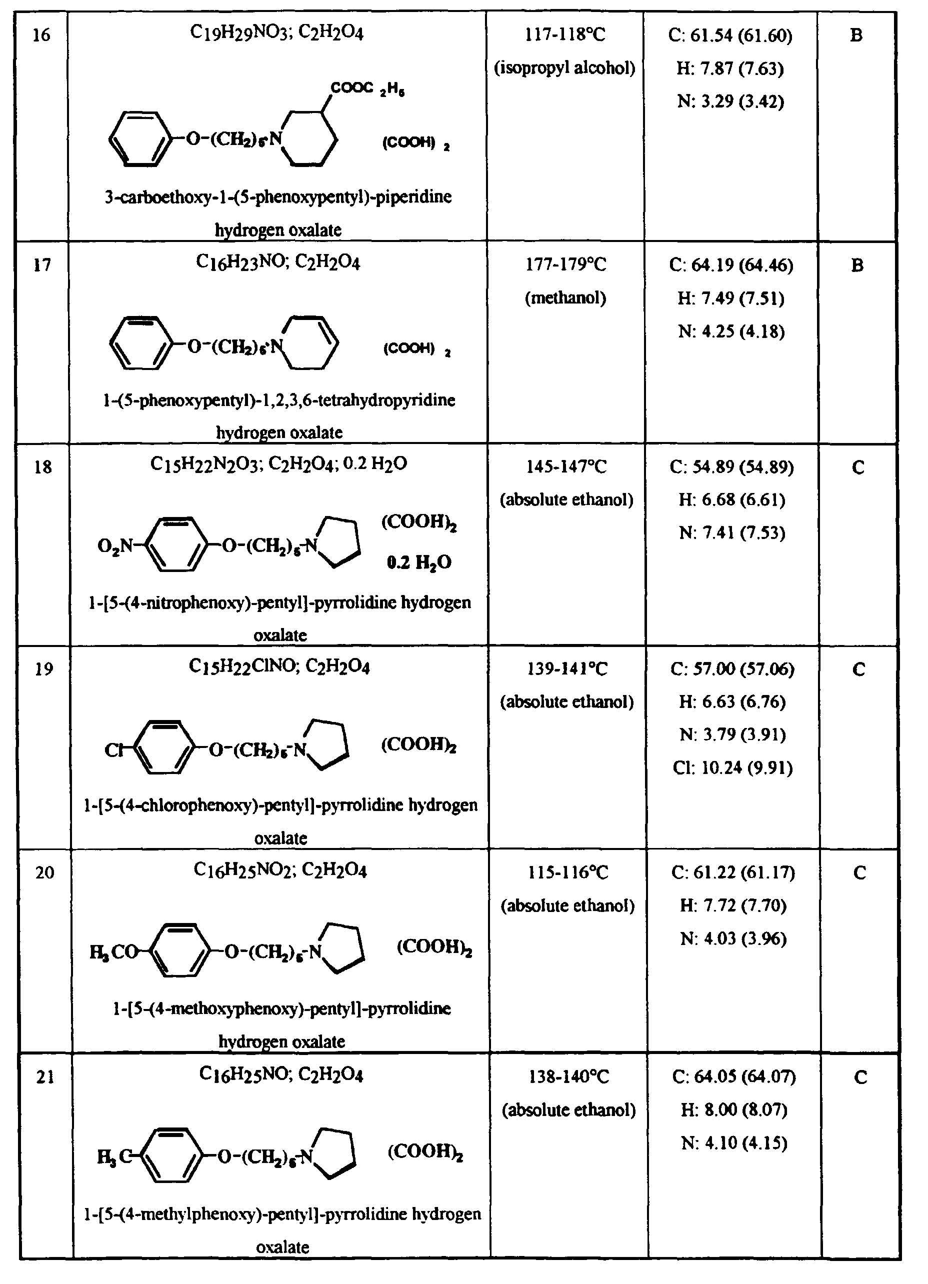

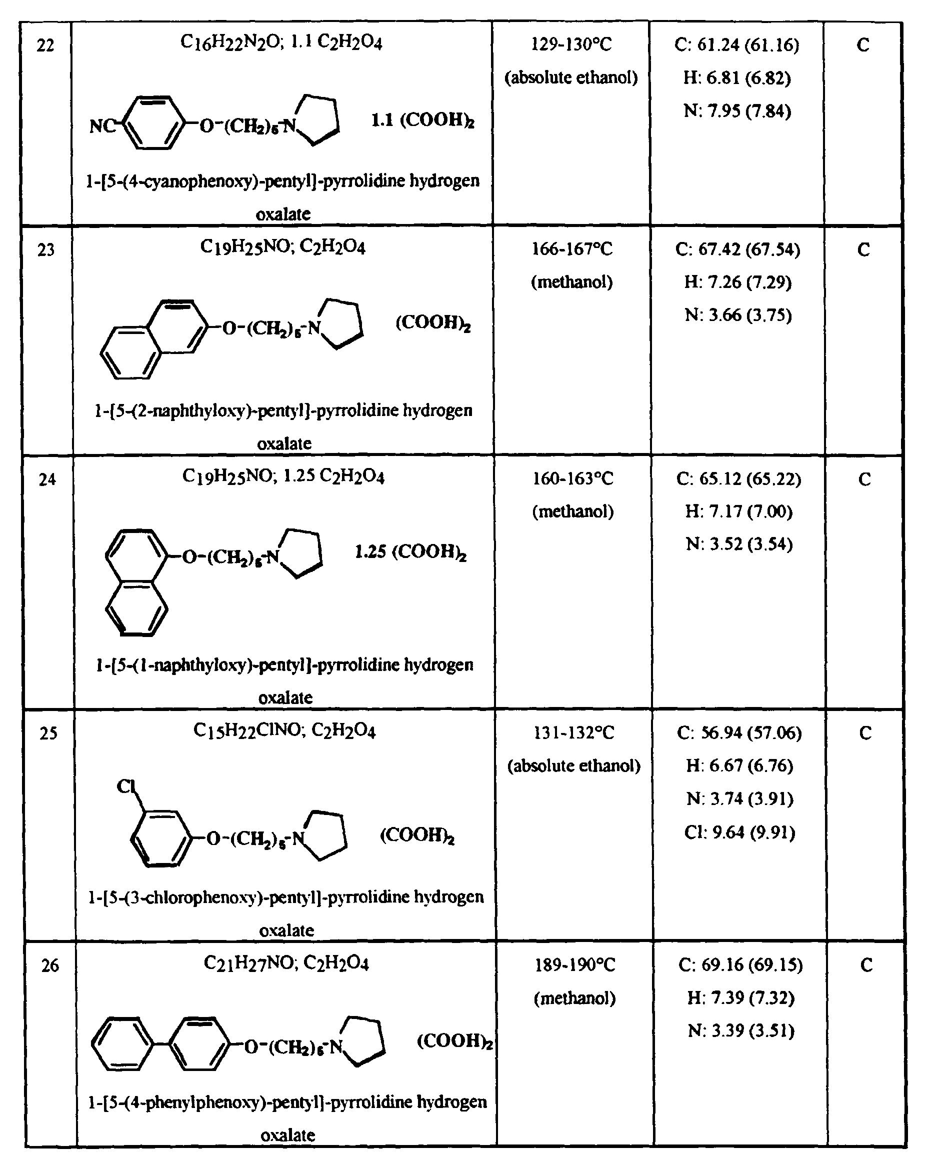

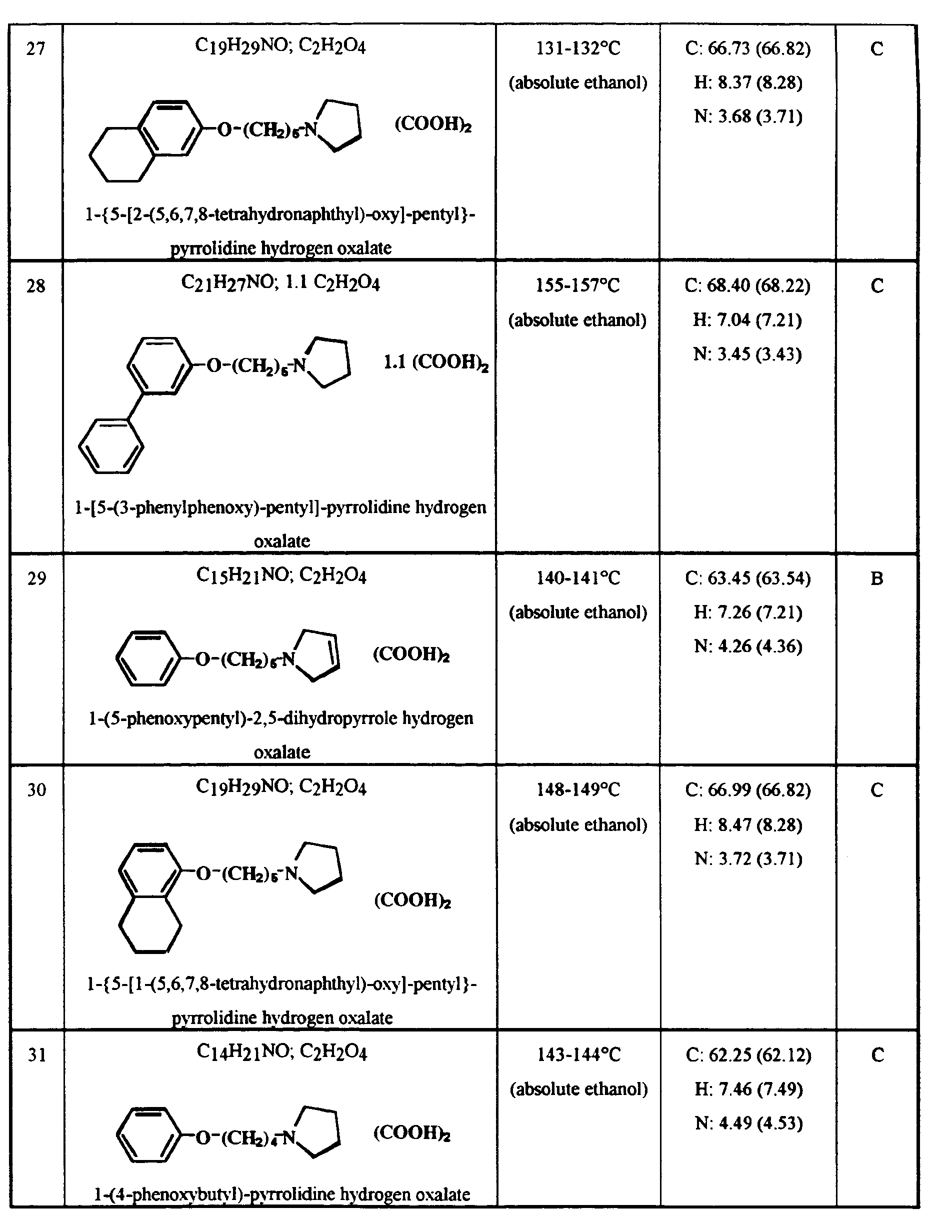

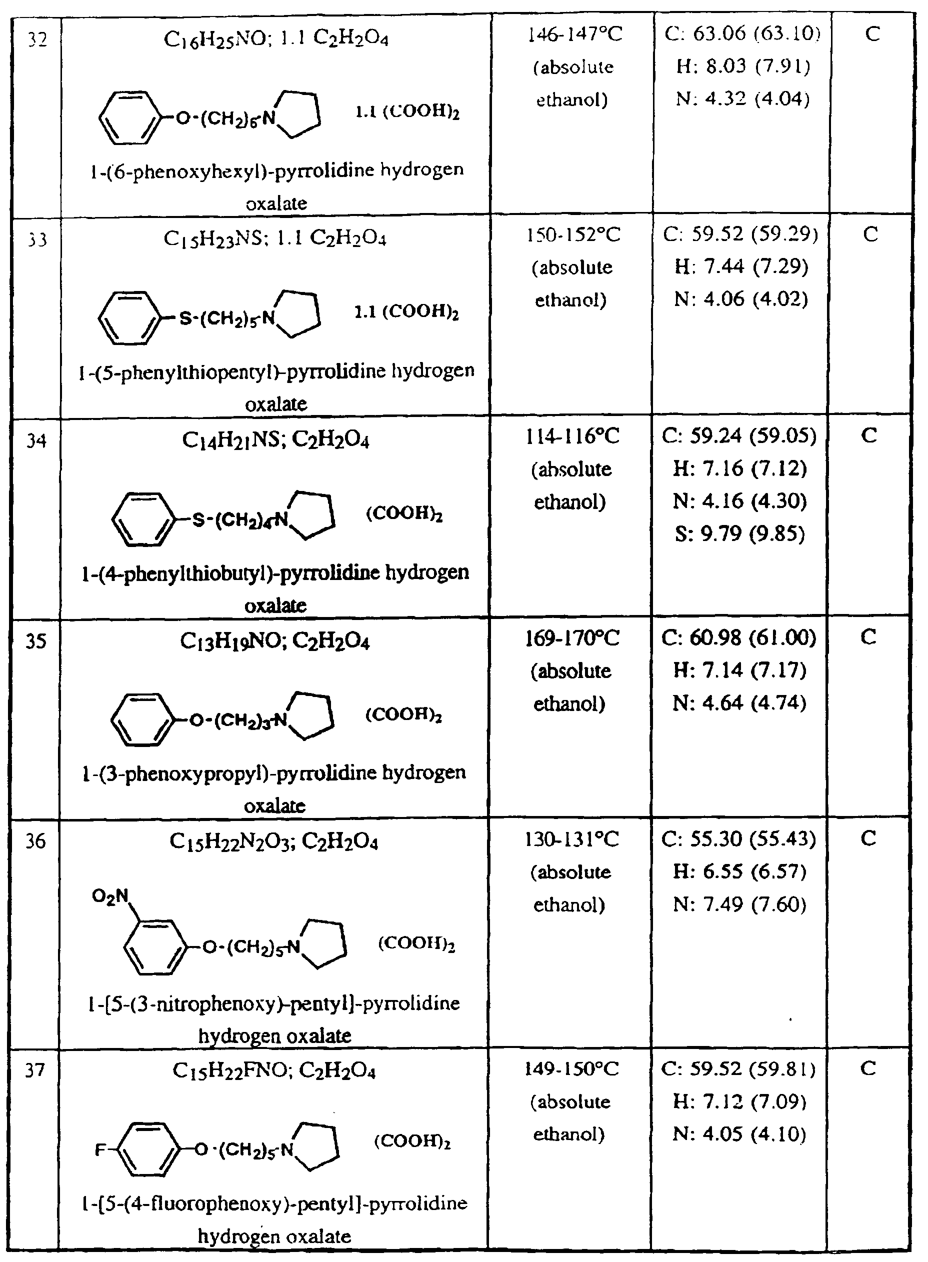

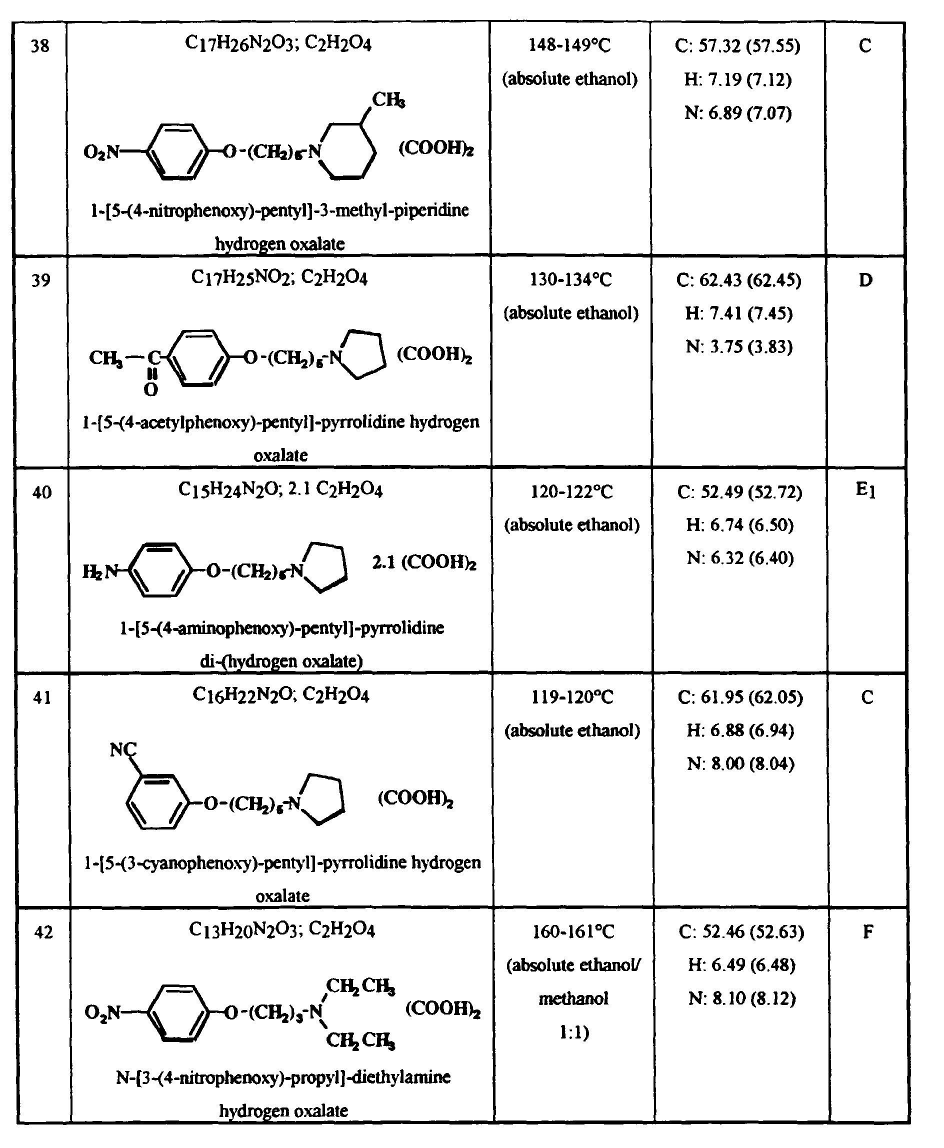

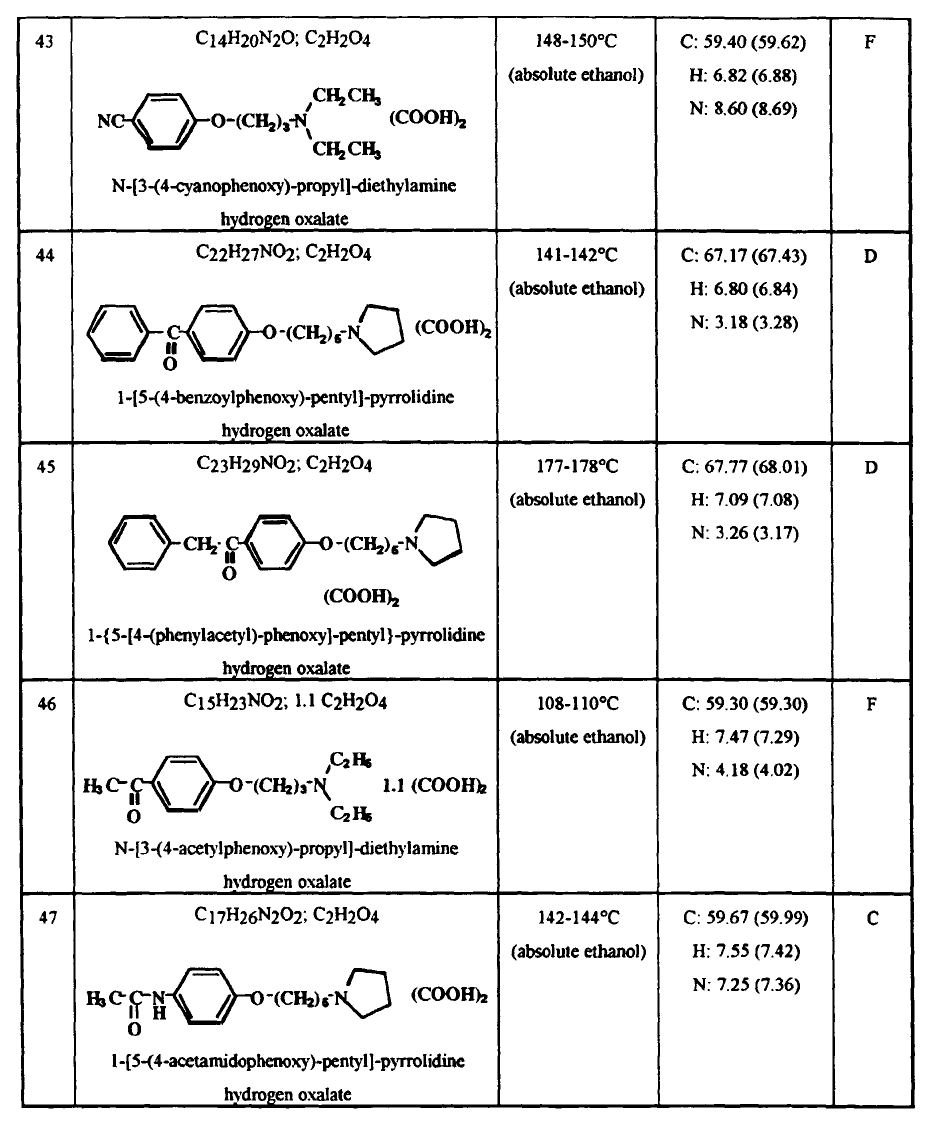

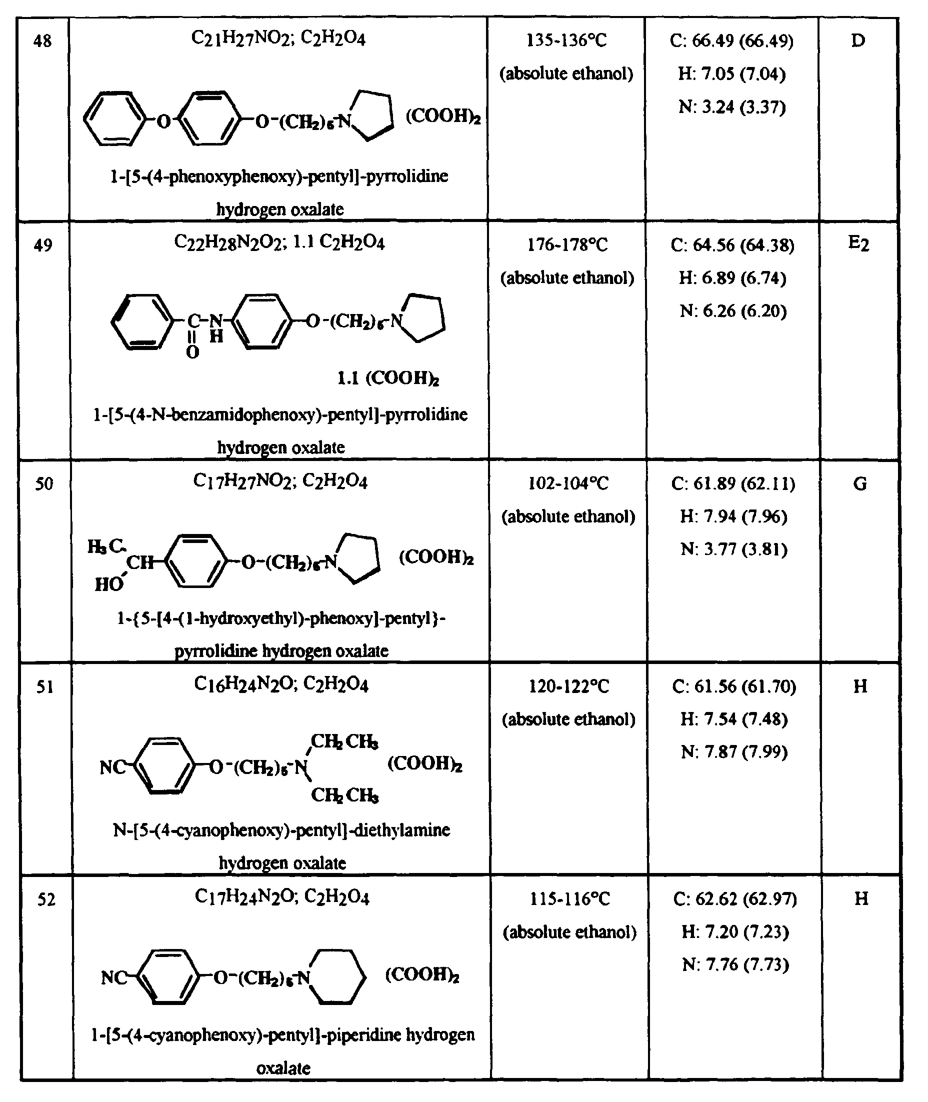

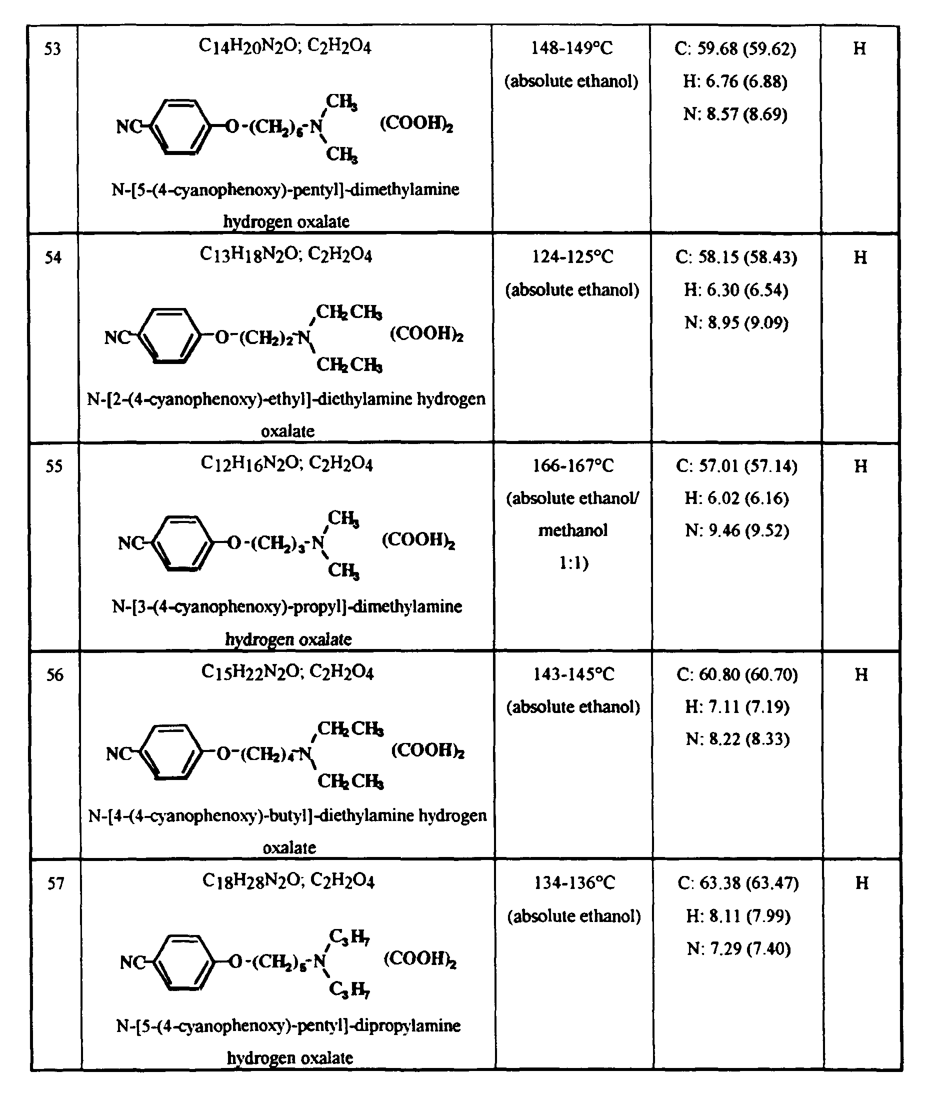

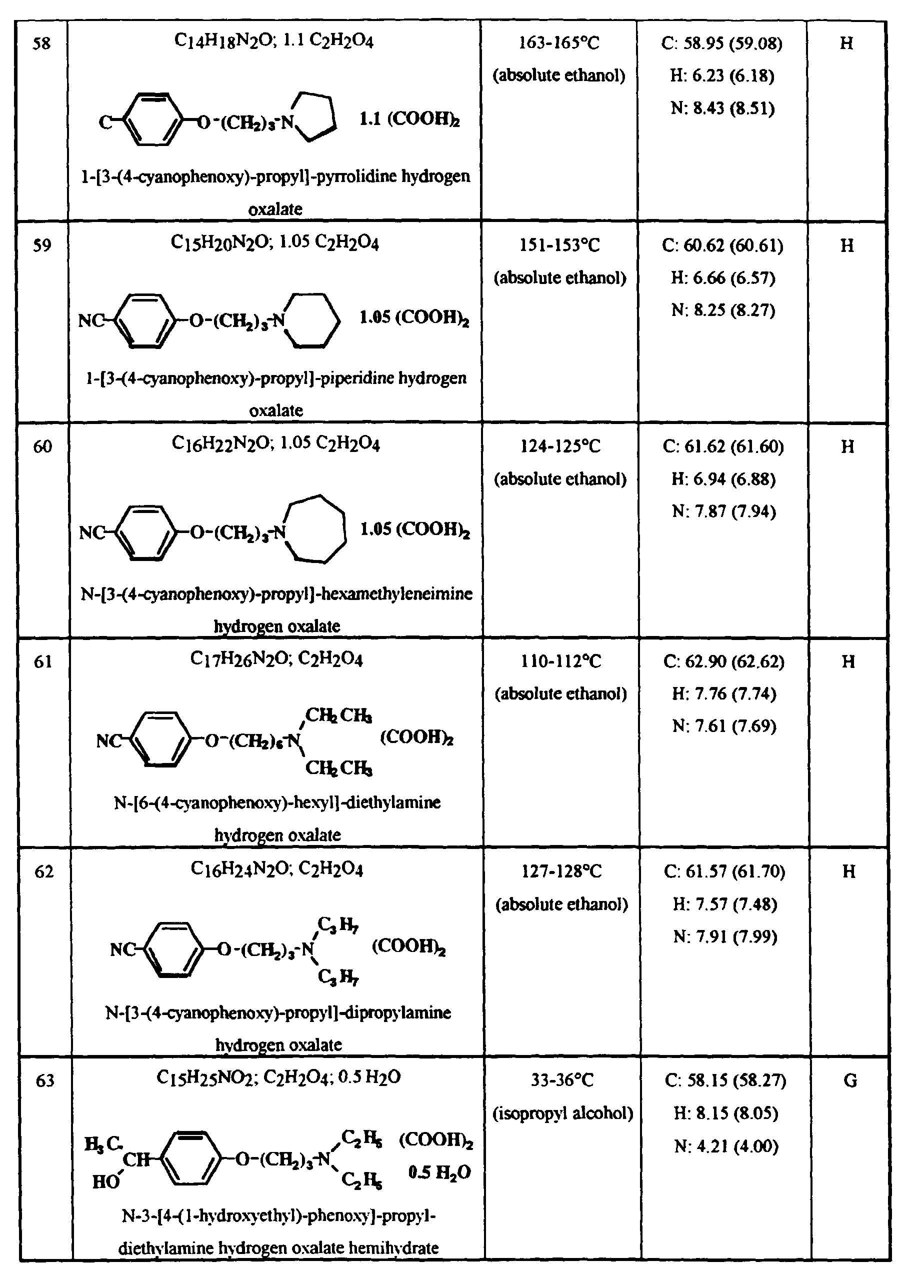

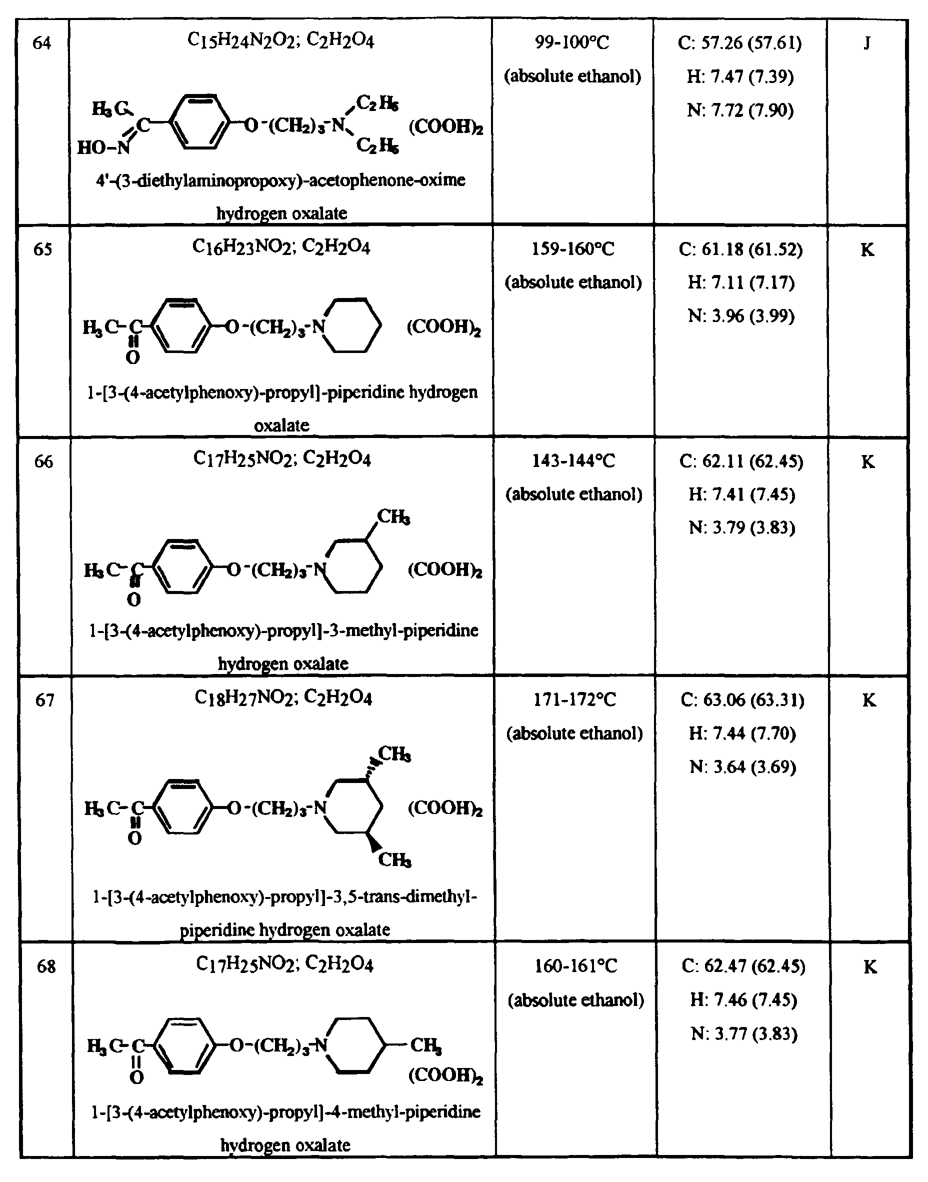

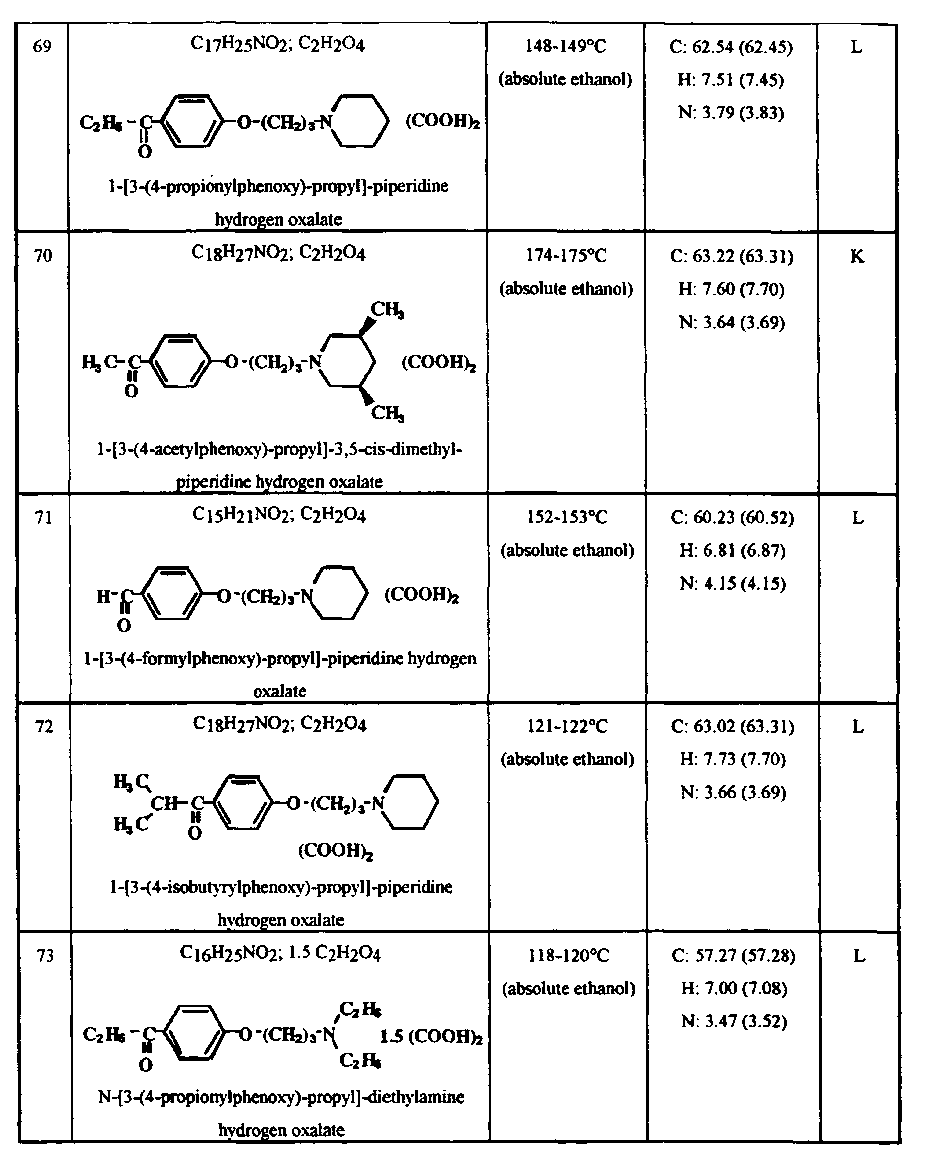

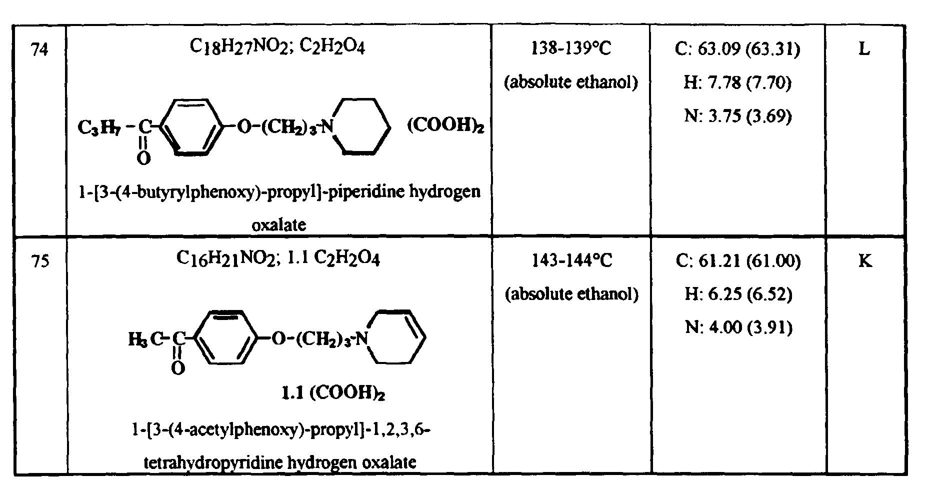

Compounds 1 to 75 are prepared according to the following procedures:

### METHOD A:

A solution of 1-bromo-5-phenoxypentane (1.4 to 3.5 mmol) in ten equivalents of the suitable secondary amine was heated to reflux temperature with stirring for 48 hours (compds. 1, 3 and 4), 24 hours (compd. 2) or 4 hours (compd. 5). After cooling, the excess base was removed under reduced pressure and the residue diluted with aqueous sodium hydroxide. The product was extracted with diethyl ether, the organic extracts washed with water, dried over magnesium sulphate, filtered and concentrated under reduced pressure. The remaining oil was converted to oxalate salt by dissolving in a small amount of absolute ethanol and adding a solution of two equivalents oxalic acid in absolute ethanol. The precipitate formed was washed with diethyl ether and recrystallised from absolute ethanol.

### METHOD B:

A solution of 1-bromo-5-phenoxypentane (0.9 to 1.7 mmol) and an excess of the suitable secondary amine (2.3 to 10 equivalents) in 10 ml absolute ethanol was heated to reflux temperature with stirring for 48 hours (compd. 6) or 24 hours (compds. 7, 8, 9, 10, 11, 12&13, 14, 15, 16, 17 and 29). After cooling, the solvent was removed under reduced pressure and the residue diluted with aqueous sodium hydroxide. The product was extracted with diethyl ether, the organic extracts washed with water, dried over magnesium sulphate, filtered and concentrated under reduced pressure. The cis and trans isomers 12 and 13 were separated by column chromatography on silica gel eluting with a solvent mixture of petroleum spirit (bp 60-80°C), diethyl ether and triethylamine in the ratio 66:33:1, and the eluent was removed under reduced pressure to leave an oil. Compounds 14 and 16 were purified by column chromatography on silica gel eluting with diethyl ether and triethylamine in the ratio 99:1, and the eluent was removed under reduced pressure to leave an oil. The oil was converted to oxalate salt (compds. 6, 7, 8, 9, 11, 12, 13, 15, 16, 17 and 29) by dissolving in a small amount of absolute ethanol and adding a solution of two equivalents of oxalic acid in absolute ethanol. If no precipitate appeared, diethyl ether was added to form a precipitate. The solid was washed with diethyl ether and recrystallised from isopropyl alcohol (compds. 6, 7, 10, 13 and 16), absolute ethanol (compds. 8, 9, 11, 12, 15 and 29) or methanol (compd. 17). The oil was converted to hydrochloride salt (compd. 14) by adding 2N HCl. The precipitate was formed in a mixture of chloroform and diethyl ether (1:1) and recrystallised from acetone.

### METHOD C:

A solution of the suitable α-bromo-ω-aryloxy alkane (0.4 to 1.4 mmol) or ω-bromoalkyl phenyl sulphide (1 mmol, compds. 33 and 34) and an excess of pyrrolidine (10 to 15 equivalents) or 3-methylpiperidine (10 equivalents, compd. 38) in 10 ml absolute ethanol was heated to reflux temperature with stirring for 24 hours or 16 hours (compd. 47). After cooling, the solvent was removed under reduced pressure and the residue diluted with aqueous sodium hydroxide. The product was extracted with diethyl ether, the organic extracts washed with water, dried over magnesium sulphate, filtered and concentrated under reduced pressure. The remaining oil was converted to oxalate salt by dissolving in a small amount of absolute ethanol and adding a solution of two equivalents oxalic acid in absolute ethanol. If no precipitate appeared, diethyl ether was added to form a precipitate. The solid was washed with diethyl ether and recrystallised from absolute ethanol.

### METHOD D:

A solution of the suitable 4'-(5-bromopentoxy)phenyl ketone (0.7 to 1 mmol, compds. 39, 44 and 45) or 1-bromo, 5-(4-phenoxyphenoxy)pentane (0.6 mmol, compd. 48) and an excess of pyrrolidine (10 to 15 equivalents) in 10 ml absolute ethanol was heated to reflux temperature with stirring for 16 hours (compds. 39, 44 and 48) or 24 hours (compd. 45). After cooling, the solvent was removed under reduced pressure and the residue diluted with aqueous sodium hydroxide. The product was extracted with chloroform (compds. 39, 45 and 48) or dichloromethane (compd. 44), the organic extracts dried over magnesium sulphate, filtered and concentrated under reduced pressure. The remaining oil was converted to oxalate salt by dissolving in a small amount of absolute ethanol and adding a solution of two equivalents oxalic acid in absolute ethanol. The precipitate was washed with diethyl ether and recrystallised from absolute ethanol (recrystallised twice from absolute ethanol in the case of compd. 39).

### METHOD E:

1. The oxalate 18 was prepared according to method C. A solution of compound 18 (0.57 mmol) in 10 ml methanol and 10 ml absolute ethanol was placed with 100 mg of palladium (5%) on carbon catalyst in a two-neck round-bottom flask fitted with a balloon filled with hydrogen. The mixture was stirred vigorously at room temperature and the flask was purged of air and filled with hydrogen. After 3 hours, the catalyst was filtered off on celite and the solvent removed under reduced pressure. The residual solid was converted to oxalate salt by dissolving in methanol and adding a solution of oxalic acid (2 equivalents) in absolute ethanol. Diethyl ether was added to form a precipitate. The product was recrystallised from absolute ethanol.
2. To a solution of compound 40 (0.35 mmol) in pyridine vigorously stirred at 0°C was added dropwise a slight excess of benzoyl chloride (0.4 mmol). The stirring was allowed to continue 20 minutes after the end of the addition after which the mixture was placed in the refrigerator overnight (16 hours). The solvent was removed under reduced pressure and the residue diluted with aqueous sodium hydroxide. The product was extracted with chloroform, the organic extracts dried over magnesium sulphate, filtered and concentrated under reduced pressure. The remaining oil was converted to oxalate salt by dissolving in a small amount of absolute ethanol and adding a solution of two equivalents oxalic acid in absolute ethanol. The precipitate was dissolved in methanol, filtered, and concentrated under reduced pressure the solid was recrystallised from absolute ethanol

### METHOD F:

In a three-neck flask kept under nitrogen was placed a solution of the suitable phenol (1.6 mmol), 3-(diethylamino)propanol (1.5 mmol), and triphenyl phosphine (1.9 mmol) in 10 ml freshly distilled tetrahydrofuran. The mixture was stirred and cooled to 0°C with an ice and salt bath. A solution of diisopropyl azodicarboxylate (2 mmol) in 10 ml tetrahydrofuran was added very slowly (typically over 40 minutes) and the mixture was allowed to warm to room temperature after which it was stirred overnight at room temperature (16 hours). The solvent was then removed under reduced pressure, the residue dissolved in ethyl acetate (20 ml) and the product extracted with 2N HCl (2x10 ml). The aqueous solution was neutralised with sodium hydroxide and the product extracted with dichloromethane. After drying over magnesium sulphate and filtration, the solvent was removed under reduced pressure. The residue was converted to oxalate salt by dissolving in a small amount of absolute ethanol and adding a solution of two equivalents oxalic acid in absolute ethanol. If no precipitate appeared, diethyl ether was added to form a precipitate. The solid was washed with diethyl ether and recrystallised from absolute ethanol (compds. 43 and 46) or from a 1:1 mixture of methanol and absolute ethanol (compd. 42).

### METHOD G:

A solution of the free base of compound 39 (0.6 mmol) or compound 46 (0.8 mmol) in 20 ml dry diethyl ether was added dropwise to a stirred suspension of lithium aluminium hydride (0.6 or 0.8 mmol) in 20 ml dry diethyl ether kept under nitrogen. The mixture was stirred at room temperature under nitrogen for two hours. Ice-cold water was carefully added and the organic layer decanted. The aqueous phase was extracted with diethyl ether. The combined organic solutions were dried over magnesium sulphate, filtered and concentrated under reduced pressure to leave a yellow oil. The oil was converted to oxalate salt by dissolving in a small amount of absolute ethanol and adding a solution of two equivalents oxalic add in absolute ethanol. The precipitate was washed with diethyl ether and recrystallised from absolute ethanol (compd 50) or from isopropyl alcohol, giving a very hygroscopic solid (compd. 63).

### METHOD H:

A solution of the suitable α-bromo-ω-(4-cyanophenoxy) alkane (0.5 to 0.7 mmol) and an excess of the suitable secondary amine (8 to 12 equivalents) in 10 ml absolute ethanol was heated to reflux temperature with stirring for 24 hours (compds. 54, 55, 57 and 60), 20 hours (compd. 52), 16 hours (compds. 56, 58, 59 and 61) or 8 hours (compd. 51) or was stirred at room temperature for 48 hours (compd. 53) or 24 hours (compd. 60). After cooling, the solvent was removed under reduced pressure and the residue diluted with aqueous sodium hydroxide. The product was extracted with diethyl ether, the organic extracts washed with water, dried over magnesium sulphate, filtered and concentrated under reduced pressure. Compound 62 was purified by column chromatography on silica gel eluting with ethyl acetate, and concentrated under reduced pressure. For all the compounds of method H, the remaining oil was converted to oxalate salt by dissolving in a small amount of absolute ethanol and adding a solution of two equivalents oxalic acid in absolute ethanol. If no precipitate appeared, diethyl ether was added to form a precipitate. The solid was washed with diethyl ether and recrystallised from absolute ethanol (two recrystallisations were required for compds. 58 and 59) or from a 1:1 mixture of methanol and absolute ethanol (compd. 55).

### METHOD J:

A solution of compound 46 (1 mmol) in 10 ml methanol was stirred at room temperature and a solution of hydroxylamine hydrochloride (2 equivalents) in 2 ml water was added. The mixture was stirred at 50-70°C in a water bath for 20 minutes. Methanol was removed under reduced pressure. The residue diluted with aqueous sodium hydroxide. The product was extracted with diethyl ether, the organic extracts washed with water, dried over magnesium sulphate, filtered and concentrated under reduced pressure. Compound 64 was purified by column chromatography on silica gel eluting with ethyl acetate, and concentrated under reduced pressure. The remaining oil was converted to oxalate salt by dissolving in a small amount of absolute ethanol and adding a solution of two equivalents oxalic acid in absolute ethanol. Diethyl ether was added to form a precipitate. The solid was washed with diethyl ether and recrystallised from absolute ethanol.

### METHOD K:

A solution of 4'-(3-bromopropoxy)acetophenone (0.8 to 1.9 mmol) and an excess of the suitable piperidine (3 to 10 equivalents) in 10 ml absolute ethanol was heated to reflux temperature with stirring for 16 hours. After cooling, the solvent was removed under reduced pressure and the residue diluted with aqueous sodium hydroxide. The product was extracted with diethyl ether, the organic extracts washed with water, dried over magnesium sulphate, filtered and concentrated under reduced pressure. The cis and trans isomers 67 and 70 were separated by column chromatography on silica gel eluting with a solvent mixture of diethyl ether, petroleum spirits (bp 60-80°C) and triethylamine in the ratio 66:33:1, and the eluent was removed under reduced pressure to leave an oil. Compound 75 was purified by column chromatography on silica gel eluting with chloroform and methanol (1:1), and concentrated under reduced pressure. The remaining oil was converted to oxalate salt by dissolving in a small amount of absolute ethanol and adding a solution of two equivalents of oxalic acid in absolute ethanol. If no precipitate appeared, diethyl ether was added to form a precipitate. The solid was washed with diethyl ether and recrystallised from absolute ethanol.

### METHOD L:

In a three-neck flask kept under nitrogen was placed a solution of the suitable 4'-hydroxyphenyl ketone (0.9 to 3 mmol), 3-(1-piperidinyl)propanol (0.9 to 3 mmol), and triphenyl phosphine (1 to 3.5 mmol) in 10 ml freshly distilled tetrahydrofuran. The mixture was stirred and cooled to 0°C with an ice and salt bath. A solution of diethyl azodicarboxylate (1 to 3.6 mmol) in 10 ml tetrahydrofuran was added very slowly (typically over 40 minutes) and the mixture was allowed to warm to room temperature after which it was stirred overnight at room temperature (16 hours). The solvent was then removed under reduced pressure, the residue dissolved in ethyl acetate (20 ml) and the product extracted with 2N HCl (2x10 ml). The aqueous solution was neutralised with sodium hydroxide and the product extracted with dichloromethane. After drying over magnesium sulphate and filtration, the solvent was removed under reduced pressure. The crude product was purified by column chromatography on silica gel eluting with diethyl ether containing 1 % triethylamine, and concentrated under reduced pressure. The residue was converted to oxalate salt by dissolving in a small amount of absolute ethanol and adding a solution of two equivalents oxalic add in absolute ethanol. If no precipitate appeared, diethyl ether was added to form a precipitate. The solid was washed with diethyl ether and recrystallised from absolute ethanol.

### Pharmacological study

Interaction of compounds with the H₃ receptor are evidenced *in vitro* by the measurement of the release of neosynthesized tritiated histamine from rat cerebral cortex synaptosomes preincubated with tritiated histidine (Garbarg et al., J. Pharmacol. Exp. Ther., 1992, 263: 304-310). The H₃ potency of compounds is measured by the progressive reversal of the tritiated histamine release inhibition by the selective H₃ agonist (R)α-methylhistamine (Arrang et al., Nature, 1987, 327: 117-123).

The effects of antagonists were estimated *in vivo* by the measurement of the tele-methylhistamine level variations in the brain of mice (Garbarg et al., J. Neurochem., 1989, 53: 1724-1730). At various time after p.o. administration of the compound, the effect of the H₃ antagonist is evidenced by the increase in the telemethylhistamine level induced. This increase is compared to the maximal effect induced by the reference H₃-antagonist thioperamide given at the dose of 10 mg/kg, p.o. This allows the calculation of the ED₅₀ value for each compound which correspond to the dose responsible for an half maximal effect.

The results are reported in the following table II:

| Ex No. | X | n | R¹R² | R³ (n₃ = 1) | Ki(nM) | ED₅₀ (mg/kg/p.o.) |
|---|---|---|---|---|---|---|
| 18 | O | 5 | -(CH₂)₄- | p-NO₂ | 39±11 | 1.1 |
| 43 | O | 3 | Et, Et | p-CN | 95±28 | 0.50 |
| 46 | O | 3 | Et, Et | p-CH₃CO | | 0.44 |
| 50 | O | 5 | -(CH₂)₄- | p-CH₃CH(OH) | | 1.0 |
| 56 | O | 4 | Et, Et | p-CN | | 1.1 |
| 59 | O | 3 | -(CH₂)₅- | p-CN | | 0.20 |
| 60 | O | 3 | -(CH₂)₆- | p-CN | | 0.64 |
| 63 | O | 3 | Et, Et | p-CH₃CH(OH) | | 0.34 |
| 64 | O | 3 | Et, Et | p-CH₃C=N(OH) | | 0.8 |
| 66 | O | 3 | -(3-Me)-(CH₂)₅- | p-CH₃CO | | 0.3 |
| 68 | O | 3 | -(4-Me)-(CH₂)₅- | p-CH₃CO | | 0.3 |
| 69 | O | 3 | -(CH₂)₅- | p-C₂H₅CO | | 0.4 |

## Claims

1. Compound of general formula (I) in which: 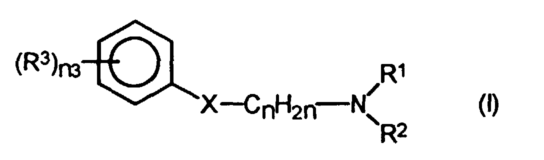
― CₙH₂ₙ is a linear or branched hydrocarbon chain with n ranging from 2 to 8;
― X is an oxygen or sulfur atom;
― R¹ and R² may be identical or different and represent each independently
• a lower alkyl or cycloalkyl, or taken together with the nitrogen atom to which they are attached,
• a saturated nitrogen-containing ring 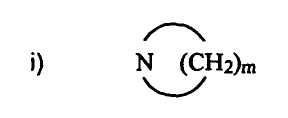 with m ranging from 4 to 7, or
• an unsaturated nitrogen-containing ring 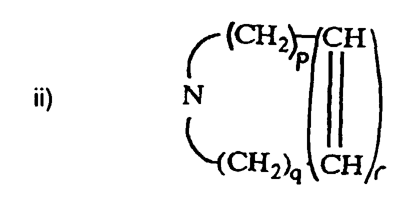 with p, q and r being 1 to 3 independently, such nitrogen-containing ring i) or ii) being unsubstituted or substituted by one or more lower alkyl or cycloalkyl, or carboalkoxy groups, or
• a morpholino group, or
• a N-substituted piperazino group: 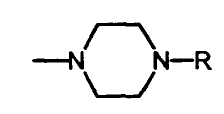 with R being a lower alkyl, an alkanoyl or an optionally substituted phenyl group;
― n₃ is an integer from 0 to 5;
― R³ represents each independently
• a halogen atom,
• a lower alkyl or cycloalkyl, a trifluoromethyl, aryl, alkoxy, aryloxy, nitro, formyl, alkanoyl, aroyl, arylalkanoyl, amino, carboxamido, cyano, alkyloximino, aryloximino, α-hydroxyalkyl, alkenyl, alkynyl, sulphamido, sulfamoyl, carboxamide, carboalkoxy, arylalkyl or oxime group,
• or taken together with the carbon atoms of the phenyl ring to which it is fused, a 5- or 6-membered saturated or unsaturated ring or a benzene ring,
as well as their pharmaceutically acceptable salts, their hydrates, their hydrated salts, the polymorphic crystalline structures of these compounds and their optical isomers, racemates, diastereoisomers and enantiomers, except compounds in which
• -NR¹R² is a pyrrolidinyl group, CₙH₂ₙ is a linear chain -(CH₂)ₙ- and n₃ is zero, X being an oxygen atom with n ranging from 3 to 5, or X being a sulfur atom with n being 4 or 5;
• -NR¹R² is a piperidinyl group, CₙH₂ₙ is a linear chain -(CH₂)ₙ- and and X is an oxygen atom, n₃ being zero with n being 2, 5 or 8 or n₃ being 1 with R³ being 4-CN and n being 5;
• -NR¹R² is a diethylamine group, X is an oxygen atom, CₙH₂ₙ is a linear chain -(CH₂)ₙ- and n₃ is 1, R³ being 4-NO₂ or 4-COCH₃ with n being 3 or R³ being 4-CN with n being 2 to 4;
• -NR¹R² is a dimethylamine group, X is an oxygen atom, CₙH₂ₙ is a linear chain -(CH₂)ₙ- and n³ is 1, R³ being 4-CN with n being 3.

2. Compound according to claim 1, in which R¹ and R² are independently a lower alkyl group.

3. Compound according to claim 2, in which R¹ and R² are each an ethyl group.

4. Compound according to claim 1, in which -NR¹R² is a saturated nitrogen-containing ring: m being as 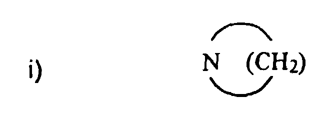 defined in claim 1.

5. Compound according to claim 4, characterized in that m is 4, 5 or 6.

6. Compound according to claim 1, characterized in that -NR¹R² is an unsaturated nitrogen-containing ring: 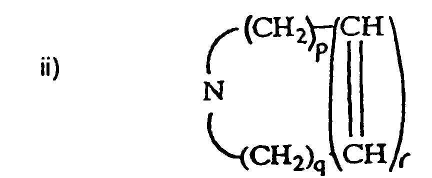 p, q and r being as defined in claim 1, preferably p, q and r are 1 or 2, more preferably p is 2 and q and r are 1.

7. Compound according to anyone of claims 4 to 6, characterized in that the nitrogen-containing ring i) or ii) is unsubstituted.

8. Compound according to anyone of claim 4 to 6, characterized in that the nitrogen-containing ring i) or ii) is substituted, preferably mono-substituted with an alkyl group.

9. Compound according to claim 8, characterized in that the nitrogen-containing ring is mono-substituted with a methyl group.

10. Compound according to claim 1, characterized in that -NR¹R² is a morpholino group.

11. Compound according to claim 1, characterized in that -NR¹R² is a N-substituted piperazino group, preferably N-acetylpiperazino.

12. Compound according to anyone of claims 1 to 11, characterized in that n₃ is zero.

13. Compound according to anyone of claims 1 to 11, characterized in that n₃ is 1 with R³ being as defined in claim 1 and preferably in para-position.

14. Compound according to anyone of claims 1 to 11 and 13, characterized in that R³ is a lower alkyl, preferably a C₁-C₄ alkyl.

15. Compound according to anyone of claims 1 to 11 and 13, characterized in that R³ is a halogen atom, a cyano, nitro, alkanoyl, alkyloximine or hydroxyalkyl, preferably CN, NO₂, COCH₃, COC₂H₅, H₃C-C=N-OH or H₃C-CHOH.

16. Compound according to anyone of claims 1 to 11, characterized in that R³ taken together with the carbon atoms of the phenyl group to which it is fused, form a 5- or 6- membered saturated or unsaturated ring, in particular a 5,6,7,8-tetrahydronaphthyl group.

17. Compound according to anyone of claims 1 to 11, characterized in that R³ taken together with the phenyl group to which it is fused, form a naphthyl group.

18. Compound according to anyone of claims 1 to 17, characterized in that -CₙH₂ₙ- is a linear hydrocarbon chain -(CH₂)ₙ-, n being as defined in claim 1.

19. Compound according to anyone of claims 1 to 18, characterized in that X is an oxygen atom.

20. Compound according to anyone of claims 1 to 18, characterized in that X is a sulfur atom.

21. Compound according to anyone of claims 1 to 20, characterized in that n is varying from 3 to 5 and is preferably 3.

22. Compound according to anyone of claims 1 to 21, characterized in that it is one of the following compounds:
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(6-phenoxyhexyl)-pyrrolidine
1-[5-(3-nitrophenoxy)-pentyl]-pyrrolidine
1-[5-(4-fluorophenoxy)-pentyl]-pyrrolidine
1-[5-(4-nitrophenoxy)-pentyl]-3-methyl-piperidine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
1-[5-(4-benzoylphenoxy)-pentyl]-pyrrolidine
1-{5-[4-(phenylacetyl)-phenoxy]-pentyl}-pyrrolidine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-(4-(1-hydroxyethyl)-phenoxy]-pentyl}-pyrrolidine
1-[5-(4-cyanophenoxy)-pentyl]-diethylamine
N-[5-(4-cyanophenoxy)-pentyl]-dimethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine

23. Compound according to anyone of claims 1 to 22, characterized in that it is one of the following compounds:
1-[5-(4-nitrophenoxy)-pentyl]-pyrrolidine
1-{5-[4-(1-hydroxyethyl)-phenoxy]-pentyl}-pyrrolidine
1-[3-(4-cyanophenoxy)-propyl]-piperidine
N-[3-(4-cyanophenoxy)-propyl]-hexamethyleneimine
N-3-[4-(1-hydroxyethyl)-phenoxy]-propyl-diethylamine
4-(3-diethylaminopropoxy)-acetophenone-oxime
1-[3-(4-acetylphenoxy)-propyl]-3-methyl-piperidine
1-[3-(4-acetylphenoxy)-propyl]-4-methyl-piperidine
1-[3-(4-propionylphenoxy)-propyl]-piperidine

24. Pharmaceutical composition characterized in that it comprises as active ingredient, a therapeutically effective amount of a compound according to anyone of claim 1 to 23 in combination with a pharmaceutically acceptable vehicle or excipient.

25. Medicament acting as an antagonist of the histamine H₃-receptors, characterized in that it comprises as active ingredient, an effective amount of a compound according to anyone of claims 1 to 23.

26. Use of a compound of general formula (I) in which: 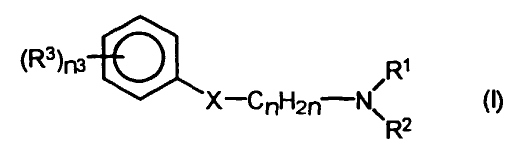
― CₙH₂ₙ is a linear or branched hydrocarbon chain with n ranging from 2 to 8;
― X is an oxygen or sulfur atom;
― R¹ and R² may be identical or different and represent each independently
• a lower alkyl or cycloalkyl, or taken together with the nitrogen atom to which they are attached,
• a saturated nitrogen-containing ring 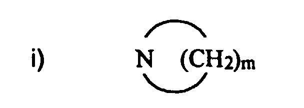 with m ranging from 4 to 7, or
• an unsaturated nitrogen-containing ring 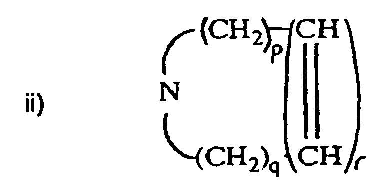 with p, q and r being 1 to 3 independently, such nitrogen-containing ring i) or ii) being unsubstituted or substituted by one or more lower alkyl or cycloalkyl, or carboalkoxy groups, or
• a morpholino group, or
• a N-substituted piperazino group: 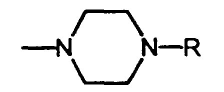 with R being a lower alkyl, an alkanoyl or an optionally substituted phenyl group;
― n₃ is an integer from 0 to 5;
― R³ represents each independently
• a halogen atom,
• a lower alkyl or cycloalkyl, a trifluoromethyl, aryl, alkoxy, aryloxy, nitro, formyl, alkanoyl, aroyl, arylalkanoyl, amino, carboxamido, cyano, alkyloximino, aryloximino, α-hydroxyalkyl, alkenyl, alkynyl, sulphamido, sulfamoyl, carboxamide, carboalkoxy, arylalkyl or oxime group,
• or taken together with the carbon atoms of the phenyl ring to which it is fused, a 5- or 6-membered saturated or unsaturated ring or a benzene ring.
as well as their pharmaceutically acceptable salts, their hydrates, their hydrated salts, the polymorphic crystalline structures of these compounds and their optical isomers, racemates, diastereoisomers and enantiomers, for the preparation of a medicament acting as an antagonist of the histamine H₃-receptors.

27. Use according to claim 26, characterized in that compound (I) is as defined in any one of claims 2 to 21.

28. Use according to claim 26 characterized in that compound (I) is one of the following compounds:
1-(5-phenoxypentyl)-piperidine
1-(5-phenoxypentyl)-pyrrolidine
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(4-phenoxybutyl)-pyrrolidine
1-(6-phenoxyhexyl)-pyrrolidine
1-(5-phenylthiopentyl)-pyrrolidine
1-(4-phenylthiobutyl)-pyrrolidine
1-(3-phenoxypropyl)-pyrrolidine
1-[5-(3-nitrophenoxy)-pentyl]-pyrrolidine
1-[5-(4-fluorophenoxy)-pentyl]-pyrrolidine
1-[5-(4-nitrophenoxy)-pentyl]-3-methyl-piperidine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
N-[3-(4-nitrophenoxy)-propyl]-diethylamine
N-[3-(4-cyanophenoxy)-propyl]-diethylamine
1-[5-(4-benzoylphenoxy)-pentyl]-pyrrolidine
1-{5-[4-(phenylacetyl)-phenoxy]-pentyl}-pyrrolidine
N-[3-(4-acetylphenoxy)-propyl]-diethylamine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-[4-(1-hydroxyethyl)-phenoxy]-pentyl}-pyrrolidine
1-[5-(4-cyanophenoxy)-pentyl]-diethylamine
1-[5-(4-cyanophenoxy)-pentyl]-piperidine
N-[5-(4-cyanophenoxy)-pentyl]-dimethylamine
N-[2-(4-cyanophenoxy)-ethyl]-diethylamine
N-[3-(4-cyanophenoxy)-propyl]-dimethylamine
N-[4-(4-cyanophenoxy)-butyl]-diethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine

29. Use according to claim 26, characterized in that compound (I) is one of the following compounds:
1-[5-(4-nitrophenoxy)-pentyl]-pyrrolidine
N-[3-(4-cyanophenoxy)-propyl]-diethylamine
N-[3-(4-acetylphenoxy)-propyl]-diethylamine
1-{5-[4-(1-hydroxyethyl)-phenoxy]-pentyl}-pyrrolidine
N-[4-(4-cyanophenoxy)-butyl]-diethylamine
1-[3-(4-cyanophenoxy)-propyl]-piperidine
N-[3-(4-cyanophenoxy)-propyl]-hexamethyleneimine
N-3-[4-(1-hydroxyethyl)-phenoxy]-propyl-diethylamine
4-(3-diethylaminopropoxy)-acetophenone-oxime
1-[3-(4-acetylphenoxy)-propyl]-3-methyl-piperidine
1-[3-(4-acetylphenoxy)-propyl]-4-methyl-piperidine
1-[3-(4-propionylphenoxy)-propyl]-piperidine

30. Medicament according to anyone of claims 25 to 29, for the treatment of central nervous system disorders, in particular Alzheimer disease, mood and attention alterations, cognitive deficits in psychiatric pathologies, obesity, vertigo and motion sickness.

31. Medicament according to anyone of claims 25 to 29, having psychotropic effects, promoting wakefulness, attention, memory and improving mood, intended to be used in particular in the treatment of Alzheimer disease and other cognitive disorders in aged persons, depressive or asthenic states.

32. Medicament according to anyone of claims 25 to 29, having nootropic effects, intended to be used in particular in treatment to stimulate attention and memorization capacity.

33. Medicament according to anyone of claims 25 to 29, for the treatment of obesity, vertigo and motion sickness.

34. Medicament according to anyone of claims 25 to 29, for the treatment of CNS disorders, in particular of aged persons.
